(19) Europäisches Patentamt / European Patent Office / Office européen des brevets

(11) **EP 4 378 522 A2**

(12) **EUROPEAN PATENT APPLICATION**
published in accordance with Art. 153(4) EPC

(43) Date of publication:
**05.06.2024 Bulletin 2024/23**

(21) Application number: **22909928.8**

(22) Date of filing: **19.12.2022**

(51) International Patent Classification (IPC):
**A61N 5/10** (2006.01)          **G01T 3/06** (2006.01)

(52) Cooperative Patent Classification (CPC):
**A61N 5/10; G01T 3/00; G01T 3/06; G01T 7/00;**
**Y02E 30/30**

(86) International application number:
**PCT/CN2022/139959**

(87) International publication number:
**WO 2023/116615 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.12.2021   CN 202111579846**

(71) Applicant: **Neuboron Therapy System Ltd.
Xiamen, Fujian 361026 (CN)**

(72) Inventors:
• **WANG, Chao
  Nanjing, Jiangsu 211112 (CN)**
• **LIU, Yuan-hao
  Nanjing, Jiangsu 211112 (CN)**

(74) Representative: **Gunzelmann, Rainer
  Wuesthoff & Wuesthoff
  Patentanwälte und Rechtsanwalt PartG mbB
  Schweigerstraße 2
  81541 München (DE)**

(54) **NEUTRON DOSE MEASUREMENT METHOD AND APPARATUS**

(57)     A neutron dose measurement method and apparatus. The neutron dose measurement method comprises: determining a unit to be corrected that corresponds to a dose monitoring system (S10); on the basis of the number of rays emitted, after neutron activation, by a nonmetal unit to be activated, and the unit to corrected that corresponds to the dose monitoring system, determining a first correction factor (S20); and correcting the dose monitoring system on the basis of the first correction factor, so as to determine a real-time neutron dose of a patient (S30). By means of the present application, a first correction factor is determined to correct the sensitivity of a dose monitoring system, so as to determine a real-time neutron dose of a patient, so as to prevent a measurement error caused by a sensitivity reduction or a position change in the dose monitoring system, thereby improving the accuracy and reliability of a measurement result.

```
                                                           ┌─────────────────────────────┐ S10
                                                           │ Determine a unit to be      │
                                                           │ corrected corresponding to  │
                                                           │ a dose monitoring system    │
                                                           └──────────────┬──────────────┘
                                                                          │
                                                           ┌──────────────▼──────────────┐ S20
                                                           │ Determine a first correction│
                                                           │ factor based on the number  │
                                                           │ of rays emitted by a nonmetal│
                                                           │ unit to be activated after  │
                                                           │ neutron activation and the  │
                                                           │ unit to be corrected        │
                                                           │ corresponding to the dose   │
                                                           │ monitoring system           │
                                                           └──────────────┬──────────────┘
                                                                          │
                                                           ┌──────────────▼──────────────┐ S30
                                                           │ Correct the dose monitoring │
                                                           │ system based on the first   │
                                                           │ correction factor to        │
                                                           │ determine a real-time       │
                                                           │ neutron dose of a patient   │
                                                           └─────────────────────────────┘
```

FIG. 1

EP 4 378 522 A2

**Description**

**TECHNICAL FIELD**

The present disclosure relates to the technical field of neutron dose measurement, and in particular to a neutron dose measurement method and apparatus.

**BACKGROUND**

As eyes of Boron Neutron Capture Therapy (BNCT), the dose monitoring system plays a very important role in the whole process of tumor treatment, which can evaluate a neutron dose irradiated to a patient and stop irradiation when the neutron dose reaches a preset value.

However, although the existing dose monitoring system can measure the real-time neutron dose of a patient, the sensitivity of the dose monitoring system decreases and there are errors in the measurement results after a period of measurement, so that it is impossible to accurately control the irradiation time according to the preset value of the neutron dose.

**SUMMARY**

The present disclosure is provided in order to solve the above technical problems. Embodiments of the present disclosure provide a neutron dose measurement method and apparatus.

In a first aspect, an embodiment of the present disclosure provides a neutron dose measurement method. The neutron dose measurement method includes: determining a unit to be corrected corresponding to a dose monitoring system; determining a first correction factor based on a number of rays emitted by a nonmetal unit to be activated after neutron activation and the unit to be corrected corresponding to the dose monitoring system, wherein the nonmetal unit to be activated includes a nonmetal component and a first detection part configured to detect the number of rays emitted by the nonmetal component after neutron activation; and correcting the dose monitoring system based on the first correction factor to determine a real-time neutron dose of a patient.

In conjunction with the first aspect, in some implementations of the first aspect, the unit to be corrected is set as a counting rate to be corrected; a first neutron reaction rate is determined based on the number of rays emitted by the nonmetal unit to be activated after neutron activation; the first correction factor is determined based on the counting rate to be corrected and the first neutron reaction rate.

In conjunction with the first aspect, in some implementations of the first aspect, a nonmetal average counting rate of an activation period corresponding to the nonmetal unit to be activated is determined based on the counting rate to be corrected; the first correction factor is determined based on the first neutron reaction rate and the nonmetal average counting rate.

In conjunction with the first aspect, in some implementations of the first aspect, a second correction factor is determined based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated includes a metal component and a second detection part configured to detect the number of rays emitted by the metal component after neutron activation; and the first correction factor is corrected based on the second correction factor to correct the corrected dose monitoring system.

In conjunction with the first aspect, in some implementations of the first aspect, a second neutron reaction rate is determined based on the number of rays emitted by the metal unit to be activated after neutron activation; a metal average counting rate of the activation period corresponding to the metal unit to be activated is determined based on the counting rate to be corrected; the second correction factor is determined based on the first neutron reaction rate and the metal average counting rate and/or the second neutron reaction rate.

In conjunction with the first aspect, in some implementations of the first aspect, correcting the first correction factor based on the second correction factor to correct the corrected dose monitoring system includes: determining difference information between the first correction factor and the second correction factor; determining a correction value corresponding to the dose monitoring system based on the first correction factor when the first correction factor is determined to meet a preset difference threshold condition based on the difference information; determining the real-time neutron dose of the patient based on the correction value corresponding to the dose monitoring system, a dose conversion factor and irradiated time corresponding to the patient.

In conjunction with the first aspect, in some implementations of the first aspect, the dose conversion factor includes a boron dose conversion factor, wherein determining the real-time neutron dose of the patient based on the correction value corresponding to the dose monitoring system, the dose conversion factor and the irradiated time corresponding to the patient includes: determining a cancer cell real-time dose-rate correction value corresponding to the dose monitoring system based on the correction value and the boron dose conversion factor; and determining the real-time neutron dose of cancer cells in the patient based on the cancer cell real-time dose-rate correction value and the irradiated time.

In conjunction with the first aspect, in some implementations of the first aspect, the dose conversion factor includes a non-cancer cell dose conversion factor, wherein determining the real-time neutron dose of the patient based on the correction value corresponding to the dose monitoring system, the dose conversion factor and the irradiated time corresponding to the patient includes: determining a non-cancer cell real-time dose-rate correction value corresponding to the dose monitoring system based on the correction value corresponding to the dose monitoring system and the non-cancer cell dose conversion factor; and determining the real-time neutron dose of non-cancer cells in the patient based on the non-cancer cell real-time dose-rate correction value and the irradiated time.

In conjunction with the first aspect, in some implementations of the first aspect, a material of the nonmetal unit to be activated includes at least one of phosphorus, sulfur, silicon and bromine.

In a second aspect, an embodiment of the present disclosure provides a neutron dose measurement apparatus. The neutron dose measurement apparatus includes a first determining module configured to determine a unit to be corrected corresponding to a dose monitoring system; a second determining module configured to determine a first correction factor based on a number of rays emitted by a nonmetal unit to be activated after neutron activation and the unit to be corrected corresponding to the dose monitoring system, wherein the nonmetal unit to be activated includes a nonmetal component and a first detection part configured to detect the number of rays emitted by the nonmetal component after neutron activation; and a third determining module configured to correct the dose monitoring system based on the first correction factor to determine a real-time neutron dose of a patient.

In conjunction with the second aspect, in some implementations of the second aspect, the second determining module is configured to: set the unit to be corrected as a counting rate to be corrected; determine a first neutron reaction rate based on the number of rays emitted by the nonmetal unit to be activated after neutron activation; and determine the first correction factor based on the counting rate to be corrected and the first neutron reaction rate.

Further, the second determining module is further configured to determine a nonmetal average counting rate of an activation period corresponding to the nonmetal unit to be activated based on the counting rate to be corrected; and determine the first correction factor based on the first neutron reaction rate and the nonmetal average counting rate.

In conjunction with the second aspect, in some implementations of the second aspect, a fourth determining module is configured to determine a second correction factor based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated includes a metal component and a second detection part configured to detect the number of rays emitted by the metal component after neutron activation. A fifth determining module is configured to correct the first correction factor based on the second correction factor to correct the corrected dose monitoring system.

Further, the second determining module is configured to determine a second neutron reaction rate based on the number of rays emitted by the metal unit to be activated after neutron activation; determine a metal average counting rate of the activation period corresponding to the metal unit to be activated based on the counting rate to be corrected; and determine the second correction factor based on the first neutron reaction rate and the metal average counting rate and/or the second neutron reaction rate.

In conjunction with the second aspect, in some implementations of the second aspect, the fifth determining module is further configured to determine difference information between the first correction factor and the second correction factor; determine a correction value corresponding to the dose monitoring system based on the first correction factor when the first correction factor is determined to meet a preset difference threshold condition based on the difference information; and determine the real-time neutron dose of the patient based on the correction value corresponding to the dose monitoring system, a dose conversion factor and irradiated time corresponding to the patient.

In a third aspect, an embodiment of the present disclosure provides a computer-readable storage medium, including a computer program for executing any of the neutron dose measurement method described above.

In a fourth aspect, an embodiment of the present disclosure provides an electronic device, including processors for executing any of the neutron dose measurement method described above and a memory for storing instructions executable by the processors.

According to the neutron dose measurement method, the neutron dose measurement apparatus, the computer-readable storage medium and the electronic device provided by the embodiment of the present disclosure, a unit to be corrected corresponding to a dose monitoring system is determined, and then a first correction factor is determined based on the number of rays emitted by a nonmetal unit to be activated after neutron activation and the unit to be corrected corresponding to the dose monitoring system. The first correction factor is used to correct the sensitivity of the dose monitoring system, so that the real-time neutron dose of the patient is determined, a measurement error caused by sensitivity reduction or position change of the dose monitoring system is avoided, and the accuracy and reliability of a measurement result are improved.

**BRIEF DESCRIPTION OF THE DRAWINGS**

The above and other objects, features and advantages of the present disclosure will become more apparent by describing the embodiments of the present disclosure in more detail with reference to accompanying drawings. The accompanying drawings are used to provide a further understanding of the embodiments of the present disclosure, and constitute a part of the specification, and serve to explain the present disclosure together with the embodiments of the present disclosure, rather than have a limitation on the present disclosure. In the accompanying drawings, the same reference numerals generally represent the same elements or steps.

FIG. 1 is a flow chart of a neutron dose measurement method according to an exemplary embodiment of the present disclosure.
FIG. 2 is a flow chart of the neutron dose measurement method according to another exemplary embodiment of the present disclosure.
FIG. 3 is a flow chart of the neutron dose measurement method according to a yet exemplary embodiment of the present disclosure.
FIG. 4 is a flow chart of the neutron dose measurement method according to another yet exemplary embodiment of the present disclosure.
FIG. 5 is a flow chart of determining a real-time neutron dose of a patient based on a correction value corresponding to a dose monitoring system, a dose conversion factor and the irradiated time corresponding to the patient according to an exemplary embodiment of the present disclosure.
FIG. 6 is a flow chart of determining the real-time neutron dose of the patient based on the correction value corresponding to the dose monitoring system, the dose conversion factor and the irradiated time corresponding to the patient according to another exemplary embodiment of the present disclosure.
FIG. 7 is a flow chart of the neutron dose measurement method according to another yet exemplary embodiment of the present disclosure.
FIG. 8 is a schematic structural diagram of a neutron dose measurement apparatus according to an exemplary embodiment of the present disclosure.
FIG. 9 is a schematic structural diagram of an electronic device according to an exemplary embodiment of the present disclosure.

**DETAILED DESCRIPTION OF THE EMBODIMENTS**

Hereinafter, the technical scheme in the embodiments of the present disclosure will be clearly and completely described with reference to the accompanying drawings in the embodiment of the present disclosure. Obviously, the described embodiments are only some of the embodiments of the present disclosure, rather than all of the embodiments. On the basis of the embodiments in the present disclosure, all other embodiments obtained by those skilled in the art without inventive efforts belong to the protection scope of the present disclosure.

Boron neutron capture therapy destroys cancer cells through nuclear reactions in tumor cells. The principle of this therapy method is as follows: first, the patient is injected with a special compound containing boron, which has a strong affinity with cancer cells. After entering the human body, the special compound quickly accumulates in cancer cells, and is rarely distributed in other tissues. This boron-containing compound is harmless to human body and has no therapeutic effect on cancer. Then, a neutron ray is used for irradiation, which does not cause significant damage to the human body. However, neutrons can have a strong nuclear reaction with boron entering cancer cells, releasing a radiation with high lethality. Such radiation has a short range with only a length of one cancer cell. Therefore, only cancer cells are killed, and surrounding tissues are not damaged. This technique of selectively killing only complex shaped cancer cells without damaging normal tissues is referred to as boron neutron capture therapy.

As eyes of boron neutron capture therapy, the dose monitoring system plays a very important role in the whole process of tumor treatment, which can evaluate a neutron dose irradiated to a patient and stop irradiation when the neutron dose reaches a preset value. In the prior art, the most accurate method for neutron dose measurement is a neutron activation method, but it is very time-consuming that the neutron activation method takes out the neutron for measurement after neutron irradiation, and the results cannot be obtained immediately, so that it is impossible to determine the real-time neutron dose of the patient. If an instantaneous activation analysis method is used, the real-time neutron dose of the patient can be determined, but the complex background of the radiation field will bring some errors in the measurement results and also result in problems such as beam disturbance. In addition, it is necessary to additionally build hardware facilities, which requires high cost and maintenance cost.

When an active detector, such as a $BF_3$ detector, is used for measurement, the neutron dose can be measured in real time, but the content of $BF_3$ used in the $BF_3$ detector will gradually decrease after neutron irradiation for a certain period of time, thereby causing the decrease in sensitivity of the $BF_3$ detector. Therefore, compared with the unused

$BF_3$ detector, the $BF_3$ detector used for a certain period of time will certainly have different responses to the same neutron intensity, which will result in errors in the measurement results. Therefore, it is necessary to correct the sensitivity of the $BF_3$ detector at regular intervals to ensure the correct operation of the dose monitoring system.

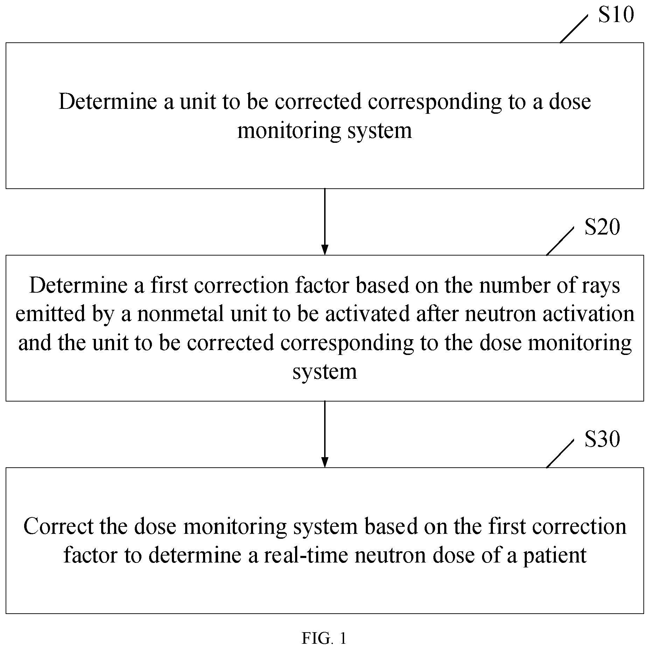

FIG. 1 is a flow chart of a neutron dose measurement method according to an exemplary embodiment of the present disclosure. As shown in FIG. 1, the neutron dose measurement method according to an embodiment of the present disclosure includes the following steps.

Step S10: a unit to be corrected corresponding to a dose monitoring system is determined.

In an embodiment of the present disclosure, the unit to be corrected includes, but is not limited to, a counting rate (in the unit of n/s), a neutron flux (in the unit of $N/cm^2$) and a neutron dose (in the unit of Gy). The counting rate can be determined based on real-time counting of a counter.

Step S20: a first correction factor is determined based on the number of rays emitted by a nonmetal unit to be activated after neutron activation and the unit to be corrected corresponding to the dose monitoring system, wherein the nonmetal unit to be activated includes a nonmetal component and a first detection part configured to detect the number of rays emitted by the nonmetal component after neutron activation.

In an embodiment of the present disclosure, the nonmetal unit to be activated may be a phosphorus flake ($^{31}P$). The first detection part can be a counter for detecting the number of rays emitted by the nonmetal component after neutron activation. For example, the nonmetal unit to be activated is placed in the air at a neutron beam outlet, or can be placed in a die body for neutron irradiation for a certain time, and $^{31}P$ will be activated into $^{31}Si$ by neutrons. After the neutron irradiation is stopped, the rays of 1266keV emitted by the nonmetal unit to be activated after neutron activation are counted by the counter, and the first correction factor is determined based on the number of rays and the unit to be corrected, wherein the first correction factor is used to correct the unit to be corrected so as to correct the sensitivity of the dose monitoring system.

Step S30: the dose monitoring system is corrected based on the first correction factor, so as to determine a real-time neutron dose of a patient.

In the practical application process, the unit to be corrected corresponding to the dose monitoring system is determined first, and then the first correction factor is determined based on the number of rays emitted by the nonmetal unit to be activated after neutron activation and the unit to be corrected. Finally, the dose monitoring system is corrected based on the first correction factor, so that the real-time neutron dose in the patient can be determined.

In the neutron dose measurement method according to the embodiment of the present disclosure, the unit to be corrected corresponding to the dose monitoring system is determined, and then the first correction factor is determined based on the number of rays emitted by the nonmetal unit to be activated after neutron activation and the unit to be corrected corresponding to the dose monitoring system. The first correction factor is used to correct the sensitivity of the dose monitoring system, so that the real-time neutron dose of the patient is determined, a measurement error caused by sensitivity reduction or position change of the dose monitoring system is avoided, and the accuracy and reliability of a measurement result are improved.

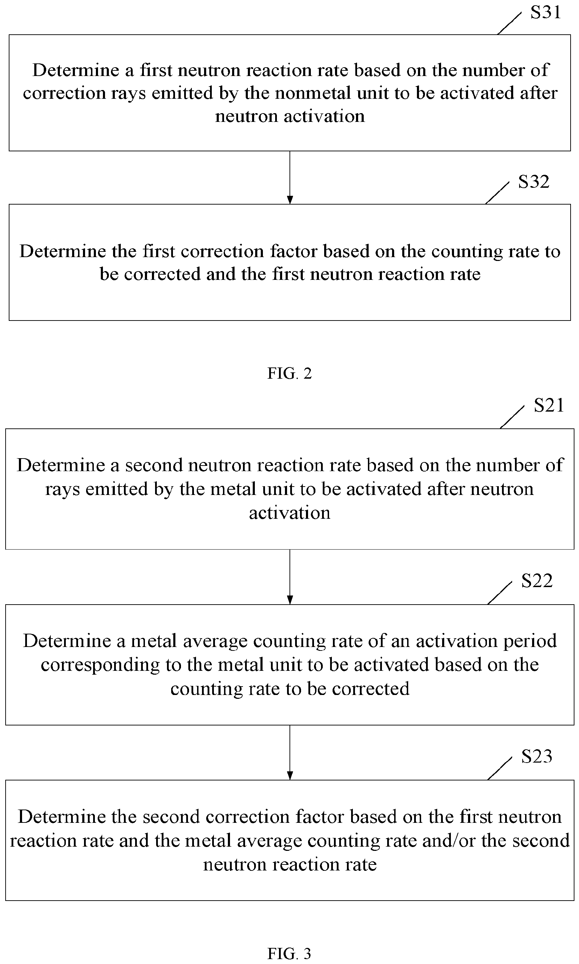

FIG. 2 is a flow chart of the neutron dose measurement method according to another exemplary embodiment of the present disclosure. The embodiment shown in FIG. 2 of the present disclosure extends from the embodiment shown in FIG. 1. The differences between the embodiment shown in FIG. 2 and the embodiment shown in FIG. 1 will be described in detail below, and the similarities will not be repeatedly described.

As shown in FIG. 2, in the neutron dose measurement method according to the embodiment of the present disclosure, the unit to be corrected is set as a counting rate to be corrected, including the following steps.

Step S31: a first neutron reaction rate is determined based on the number of correction rays emitted by the nonmetal unit to be activated after neutron activation.

In an embodiment of the present disclosure, the first neutron reaction rate $RR_1$ is determined according to the following formula (1).

$$RR_1 = \frac{\lambda \times C}{N_1 \times \varepsilon \times Y \times f_1 \times G \times (1 \quad e^{\lambda t_{irr}}) \times e^{\lambda t_c} \times (1 \quad e^{\lambda t_m})} \quad (1)$$

In the above formula (1), $\lambda$ is a decay constant, $C$ is the number of rays measured in counting time (net counting), $N_1$ is the number of target nuclei of the irradiated nonmetal unit to be activated, $\varepsilon$ is a detection efficiency of an activation detector on the correction rays, $Y$ is a correction ray branch ratio of the correction rays, $f_1$ is a self-absorption correction factor of the correction rays, $G$ is a flux fluctuation correction factor, $t_{irr}$ is neutron irradiation time, $t_c$ is cooling time (i. e., the time from the end of neutron irradiation to the beginning of measuring the number of rays), and $t_m$ is time for measuring the number of rays.

Step S32: the first correction factor is determined based on the counting rate to be corrected and the first neutron reaction rate.

For example, after the counting rate to be corrected and the first neutron reaction rate are determined, the counting rate to be corrected and the first neutron reaction rate can be calculated to obtain the first correction factor.

In an embodiment of the present disclosure, first, a nonmetal average counting rate of an activation period corresponding to the nonmetal unit to be activated is determined based on the counting rate to be corrected, and then the first correction factor is determined based on the first neutron reaction rate and the nonmetal average counting rate.

Specifically, the nonmetal average counting rate $\overline{B}_1$ is determined according to the following formula (2):

$$\overline{B}_1 = \frac{\sum\limits_{0}^{T_1} B_t}{T_1} \qquad (2)$$

In the above formula (2), $B_t$ is the counting rate to be corrected, and $T_1$ is the activation period corresponding to the nonmetal unit to be activated. The first correction factor $k_1$ is determined according to the above formula (1) and the following formula (3):

$$k_1 = \frac{RR_1}{\overline{B}_1} \qquad (3)$$

In the neutron dose measurement method according to the embodiment of the present disclosure, the first correction factor is determined by the first neutron reaction rate and the nonmetal average counting rate, so that the accuracy of the correction result is improved.

In an embodiment of the present disclosure, a second correction factor is determined based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated includes a metal component and a second detection part configured to detect the number of rays emitted by the metal component after neutron activation; and the first correction factor is corrected based on the second correction factor so as to correct the corrected dose monitoring system.

Specifically, the second correction factor can be determined based on the number of rays emitted by the nonmetal unit to be activated after neutron activation and the unit to be corrected corresponding to the dose monitoring system, or based on the number of rays emitted by the nonmetal unit to be activated after neutron activation and the number of rays emitted by the metal unit to be activated after neutron activation. The present disclosure is not specifically limited thereto.

In an embodiment of the present disclosure, the metal unit to be activated can be a gold flake. For example, the metal unit to be activated is placed in the air at the neutron beam outlet, or can be placed in the die body for neutron irradiation for a certain time, and the metal unit to be activated will generate activation reaction with neutrons. After the neutron irradiation is stopped, the rays emitted by the metal unit to be activated after neutron activation are counted by the counter, and the second correction factor is determined based on the number of rays emitted by the nonmetal unit to be activated after neutron activation and the number of rays emitted by the metal unit to be activated after neutron activation. The second correction factor is used to determine whether the correction method of the metal activation method or the nonmetal activation method is accurate.

FIG. 3 is a flow chart of the neutron dose measurement method according to another exemplary embodiment of the present disclosure. The embodiment shown in FIG. 3 of the present disclosure extends from the embodiment shown in FIG. 1. The differences between the embodiment shown in FIG. 3 and the embodiment shown in FIG. 1 will be described in detail below, and the similarities will not be repeatedly described.

As shown in FIG. 3, the neutron dose measurement method according to the embodiment of the present disclosure includes the following steps.

Step S21: a second neutron reaction rate is determined based on the number of rays emitted by the metal unit to be activated after neutron activation.

In an embodiment of the present disclosure, the second neutron reaction rate $RR_2$ is determined according to the following formula (4):

$$RR_2 = \frac{\lambda \times C}{N_2 \times \varepsilon \times Y \times f_1 \times G \times (1 - e^{-\lambda t_{irr}}) \times e^{-\lambda t_c} \times (1 - e^{-\lambda t_m})} \qquad (4)$$

In the above formula (4), $\lambda$ is the decay constant, $C$ is the number of rays measured in counting time (net counting), $N_2$ is the number of target nuclei of the irradiated metal unit to be activated, $\varepsilon$ is the detection efficiency of the activation detector on the correction rays, $Y$ is the correction ray branch ratio of the correction rays, $f_1$ is the self-absorption correction factor of the correction rays, $G$ is the flux fluctuation correction factor, $t_{irr}$ is the neutron irradiation time, $t_c$ is the cooling time (i. e., the time from the end of neutron irradiation to the beginning of measuring the number of rays), and $t_m$ is the time for measuring the number of rays.

Step S22: a metal average counting rate of the activation period corresponding to the metal unit to be activated is determined based on the counting rate to be corrected.

In an embodiment of the present disclosure, the metal average counting rate $B_2$ is determined according to the following formula (5):

$$\overline{B_2} = \frac{\sum_{0}^{T_2} B_t}{T_2} \qquad (5)$$

In the above formula (5), $B_t$ is the counting rate to be corrected, and $T_2$ is the activation period corresponding to the metal unit to be activated.

Step S23: the second correction factor is determined based on the first neutron reaction rate and the metal average counting rate and/or the second neutron reaction rate.

In an embodiment of the present disclosure, the second correction factor $k_2$ is determined according to the following formula (6).

$$k_2 = \frac{RR_2}{\overline{B_2}} \qquad (6)$$

In other embodiments of the present disclosure, the second correction factor can also be determined according to the ratio of the first neutron reaction rate to the second neutron reaction rate.

It should be noted that the manner in which the second correction factor is determined by the first neutron reaction rate and the metal average counting rate and/or the second neutron reaction rate is beneficial to improving the accuracy of the correction result.

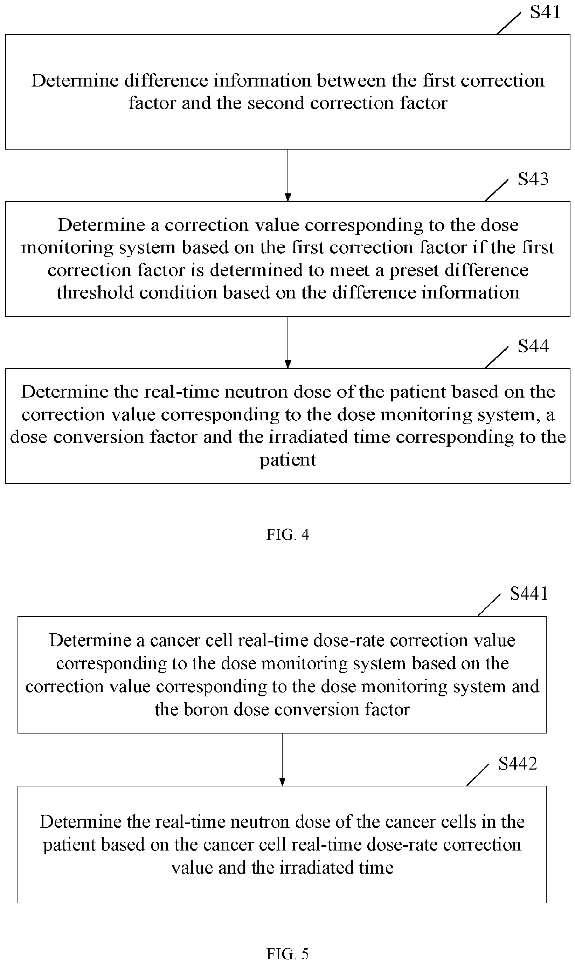

FIG. 4 is a flow chart of the neutron dose measurement method according to another exemplary embodiment of the present disclosure. The embodiment shown in FIG. 4 of the present disclosure extends from the embodiment shown in FIG. 1. The differences between the embodiment shown in FIG. 4 and the embodiment shown in FIG. 1 will be described in detail below, and the similarities will not be repeatedly described.

As shown in FIG. 4, in the neutron dose measurement method according to the embodiment of the present disclosure, correcting the first correction factor based on the second correction factor so as to correct the corrected dose monitoring system includes the following steps.

Step S41: difference information between the first correction factor and the second correction factor is determined.

In an embodiment of the present disclosure, the difference information may be a difference between the first correction factor and the second correction factor.

Step S43: a correction value corresponding to the dose monitoring system is determined based on the first correction factor if it is determined that the first correction factor meets a preset difference threshold condition based on the difference information.

In an embodiment of the present disclosure, the preset difference threshold condition can be 5% or 10% of the first correction factor, and specific value of the preset difference threshold condition can be set according to the actual situation, which is not further limited in this embodiment. If the difference between the first correction factor and the second correction factor is less than or equal to the preset difference threshold condition, it is determined that the first correction factor meets the preset difference threshold condition, and then the correction value corresponding to the dose monitoring system is determined based on a product of the first correction factor and the counting rate to be corrected according to the following formula (7):

$$B_r = B_t \times k_1 \qquad (7)$$

In the above formula (7), $B_r$ is the correction value corresponding to the dose monitoring system.

Step S44: the real-time neutron dose of the patient is determined based on the correction value corresponding to the dose monitoring system, a dose conversion factor and irradiated time corresponding to the patient.

In an embodiment of the present disclosure, the real-time neutron dose of the patient is determined based on the product of the correction value corresponding to the dose monitoring system, the dose conversion factor and the irradiated time corresponding to the patient.

In the neutron dose measurement method according to the embodiment of the present disclosure, it is verified whether the difference information between the first correction factor and the second correction factor meets the preset difference threshold condition. If so, the correction value corresponding to the dose monitoring system is obtained based on the first correction factor, so that the accuracy and reliability of the correction result of the first correction factor are improved.

FIG. 5 is a flow chart of determining the real-time neutron dose of the patient based on the correction value corresponding to the dose monitoring system, the dose conversion factor and the irradiated time corresponding to the patient according to an exemplary embodiment of the present disclosure. The embodiment shown in FIG. 5 of the present disclosure extends from the embodiment shown in FIG. 4. The differences between the embodiment shown in FIG. 5 and the embodiment shown in FIG. 4 will be described in detail below, and the similarities will not be repeatedly described.

As shown in FIG. 5, in the neutron dose measurement method according to the embodiment of the present disclosure, the step of determining the real-time neutron dose of the patient based on the correction value corresponding to the dose monitoring system, the dose conversion factor and the irradiated time corresponding to the patient includes the following steps.

Specifically, the dose conversion factor includes a boron dose conversion factor.

Step S441: a cancer cell real-time dose-rate correction value corresponding to the dose monitoring system is determined based on the correction value corresponding to the dose monitoring system and the boron dose conversion factor.

In an embodiment of the present disclosure, the cancer cell real-time dose-rate correction value corresponding to the dose monitoring system is determined based on the product of the correction value corresponding to the dose monitoring system and the boron dose conversion factor according to the following formula (8):

$$D_{t1} = \frac{B_r}{\sigma \times f_2} \times K \times N \times CBE \qquad (8)$$

In the above formula (8), $D_{t1}$ is the cancer cell real-time dose-rate correction value (in the unit of Gy / s), $\sigma$ is a thermal neutron reaction cross section (in the unit of $cm^2$), $f_2$ is a neutron attenuation correction factor caused by the activation detector, $K$ is the boron dose conversion factor of boron concentration when the flux reaches 1 $ppm$ (in the unit of $Gy \times cm^2/ppm$), $N$ is an actual boron concentration (in the unit of $ppm$), and $CBE$ is a composite biological effect factor.

Step S442: the real-time neutron dose of the cancer cells in the patient is determined based on the cancer cell real-time dose-rate correction value and the irradiated time.

In an embodiment of the present disclosure, the real-time neutron dose of the cancer cells in the patient is determined based on an integral of the cancer cell real-time dose-rate correction value in the irradiated time period according to the following formula (9).

$$D_{acm1} = \sum_{t=1}^{T} D_{t1} \qquad (9)$$

In the above formula (9), $D_{acm1}$ is a cumulative neutron dose (i.e. the real-time neutron dose) of the cancer cells in the patient over the irradiated time period, and $T$ is the irradiated time of the patient.

The neutron dose measurement method according to the embodiment of the present disclosure obtains the real-time neutron dose of the cancer cells in the patient, accurately evaluates the neutron dose irradiated into the cancer cells of the patient, and stops irradiation in time when the neutron dose reaches the preset value.

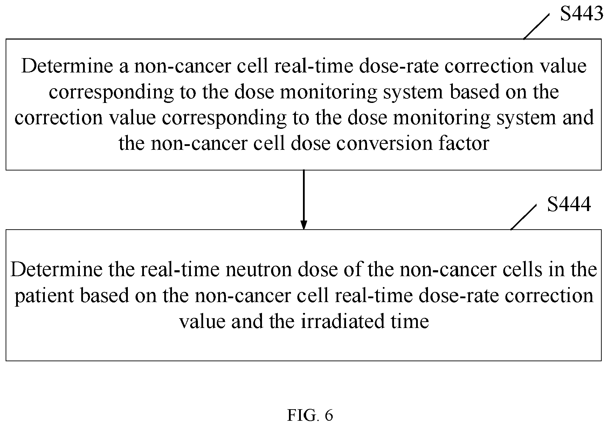

FIG. 6 is a flow chart of determining the real-time neutron dose of the patient based on the correction value corresponding to the dose monitoring system, the dose conversion factor and the irradiated time corresponding to the patient according to another exemplary embodiment of the present disclosure. The embodiment shown in FIG. 6 of the present disclosure extends from the embodiment shown in FIG. 5. The differences between the embodiment shown in FIG. 6 and the embodiment shown in FIG. 5 will be described in detail below, and the similarities will not be repeatedly described.

As shown in FIG. 6, in the neutron dose measurement method according to the embodiment of the present disclosure, the step of determining the real-time neutron dose of the patient based on the correction value corresponding to the dose monitoring system, the dose conversion factor and the irradiated time corresponding to the patient includes the following steps.

Specifically, the dose conversion factor includes a non-cancer cell dose conversion factor.

Step S443: a non-cancer cell real-time dose-rate correction value corresponding to the dose monitoring system is determined based on the correction value corresponding to the dose monitoring system and the non-cancer cell dose conversion factor.

In an embodiment of the present disclosure, the non-cancer cell real-time dose-rate correction value corresponding to the dose monitoring system is determined based on a product of the correction value corresponding to the dose monitoring system and the non-cancer cell dose conversion factor according to the following formula (10):

$$D_{t2} = \frac{B_r}{\sigma \times f_2} \times K_t \times RBE \qquad (10)$$

In the above formula (10), $D_{t2}$ is the non-cancer cell real-time dose-rate correction value (in the unit of $Gy / s$), $\sigma$ is the thermal neutron reaction cross section (in the unit of $cm^2$), $f_2$ is the neutron attenuation correction factor caused by the activation detector, $K_t$ is the non-cancer cell dose conversion factor, and RBE is a relative biological effect factor.

Step S444: the real-time neutron dose of the non-cancer cells in the patient is determined based on the non-cancer cell real-time dose-rate correction value and the irradiated time.

In an embodiment of the present disclosure, the real-time neutron dose of the non-cancer cells in the patient is determined based on an integral of the non-cancer cell real-time dose-rate correction value in the irradiated time period according to the following formula (11).

$$D_{acm2} = \sum_{t=1}^{T} D_{t2} \qquad (11)$$

In the above formula (11), $D_{acm2}$ is a cumulative neutron dose (i.e. the real-time neutron dose) of the non-cancer cells in the patient over the irradiated time period, and $T$ is the irradiated time of the patient.

In the practical application process, first, the non-cancer cell real-time dose-rate correction value corresponding to the dose monitoring system is determined based on the correction value corresponding to the dose monitoring system and the non-cancer cell dose conversion factor, and then the real-time neutron dose of the non-cancer cells in the patient is determined based on the non-cancer cell real-time dose-rate correction value and the irradiated time.

The neutron dose measurement method according to the embodiment of the present disclosure obtains the real-time neutron dose of the non-cancer cells in the patient, accurately evaluates the neutron dose irradiated into the non-cancer cells of the patient, and provides more reference data for tumor treatment.

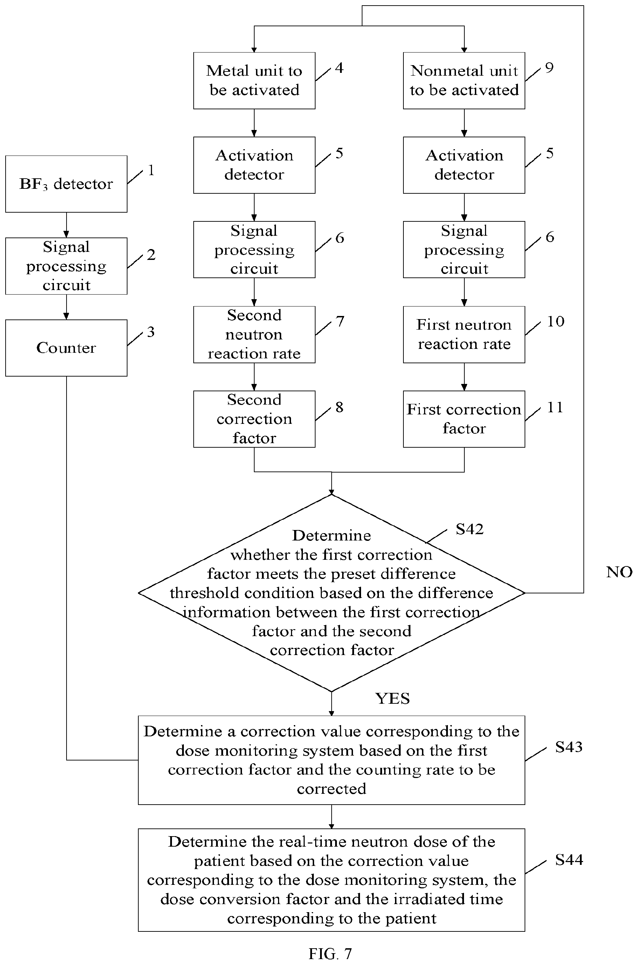

FIG. 7 is a flow chart of the neutron dose measurement method according to another exemplary embodiment of the present disclosure. The embodiment shown in FIG. 7 of the present disclosure extends from the embodiment shown in FIG. 4. The differences between the embodiment shown in FIG. 7 and the embodiment shown in FIG. 4 will be described in detail below, and the similarities will not be repeatedly described.

As shown in FIG. 7, in the neutron dose measurement method according to the embodiment of the present disclosure, prior to the step of determining the correction value corresponding to the dose monitoring system based on the first correction factor and the counting rate to be corrected if the first correction factor is determined to meet the preset difference threshold condition based on the difference information, the method further includes the following steps.

Step S42: it is determined whether the first correction factor meets the preset difference threshold condition based on the difference information between the first correction factor and the second correction factor.

In an embodiment of the present disclosure, if the difference between the second correction factor 8 and the first correction factor 11 is less than or equal to the preset difference threshold condition, the second correction factor 8 is determined to meet the preset difference threshold condition, and then Steps S43 and S44 are executed. If the difference between the second correction factor 8 and the first correction factor 11 is greater than the preset difference threshold condition, the second correction factor 8 is determined not to meet the preset difference threshold condition, and then the second correction factor 8 is obtained again by neutron activation from the metal unit 4 to be activated and the first correction factor 11 is obtained again by neutron activation from the nonmetal unit 9 to be activated, until the second correction factor 8 meets the preset difference threshold condition.

For example, assuming that the preset difference threshold condition is 10% and the difference between the second correction factor 8 and the first correction factor 11 is 7%, the difference between the second correction factor 8 and the first correction factor 11 is less than the preset difference threshold condition, and the second correction factor 8 is determined to meet the preset difference threshold condition. On the contrary, assuming that the difference between the second correction factor 8 and the first correction factor 11 is 13%, the difference between the second correction factor 8 and the first correction factor 11 is greater than the preset difference threshold condition, and the second correction factor 8 is determined not to meet the preset difference threshold condition.

As shown in FIG. 7, the $BF_3$ detector 1 is placed in a Beam Shaping Assembly (BSA) to receive neutron irradiation. The boron element in the $BF_3$ detector 1 has a nuclear reaction with neutrons to generate $^{10}B$ $(N,A)^7Li$ . Charged particles $^7Li$ and A are collected by a high voltage electrode under the drive of voltage, so as to generate an induced electric pulse signal. The pulse signal is transmitted to a signal processing circuit 2 through a coaxial cable. The signal processing circuit 2 performs pulse amplification, filtering and shaping operations on the pulse signal. The processed pulse signal is transmitted to a counter 3 for pulse counting to obtain the counting rate (i.e. the counting rate to be corrected), and the neutron beam intensity can be measured in real time by the counting rate.

However, after the boron element in the $BF_3$ detector 1 has a nuclear reaction with neutrons with a certain flux, the boron element content will gradually decrease, resulting in the decrease of the sensitivity of the $BF_3$ detector 1. Therefore, it is necessary to correct the sensitivity of the $BF_3$ detector at regular intervals to ensure the correct operation of the dose monitoring system.

The specific steps of the correction operation are as follows.

The nonmetal unit to be activated 9 is placed in the air at the neutron beam outlet, or can be placed in the die body for neutron irradiation for a certain time. The nonmetal unit to be activated 9 will have an activation reaction with neutrons. After the neutron irradiation is stopped, the activated nonmetal unit is placed in front of the activation detector 5 in the measurement apparatus, and the signal processing circuit 6 performs preprocessing operation on the correction rays emitted by the nonmetal unit to be activated 9 after neutron activation. The correction rays are shaped, noise is eliminated, and then the correction rays are counted by the counter 3 to obtain the first neutron reaction rate 10. Moreover, the first correction factor 11 is determined based on the first neutron reaction rate 10.

Further, the metal unit to be activated 4 is placed in the air at the neutron beam outlet, or can be placed in the die body for neutron irradiation for a certain time. The metal unit to be activated 4 will have an activation reaction with neutrons. After the neutron irradiation is stopped, the activated metal unit is placed in front of the activation detector 5 in the measurement apparatus, and the signal processing circuit 6 performs preprocessing operation on the correction rays emitted by the metal unit to be activated 4 after neutron activation. The correction rays are shaped, noise is eliminated, and then the correction rays are counted by the counter 3 to obtain the second neutron reaction rate 7. Moreover, the second correction factor 8 is determined based on the second neutron reaction rate 7.

It should be noted that the material of the nonmetal unit to be activated includes at least one of phosphorus, sulfur, silicon and bromine. The correction rays emitted by the metal unit to be activated and the non-metallic unit to be activated after activation include gamma rays and/or electron rays, and the activation detector includes at least one of high-purity germanium, a semiconductor detector, a scintillator and an ionization chamber detector.

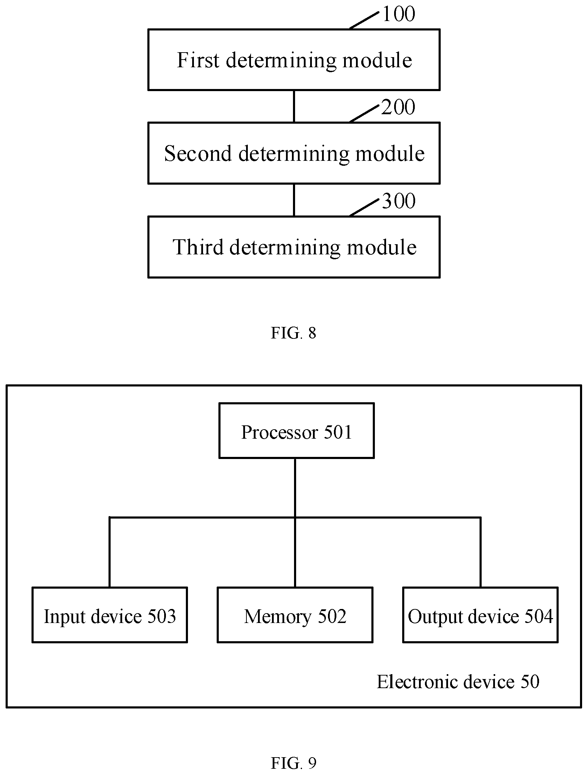

FIG. 8 is a schematic structural diagram of a neutron dose measurement apparatus according to an exemplary embodiment of the present disclosure. As shown in FIG. 8, the neutron dose measurement apparatus according to the embodiment of the present disclosure includes:

a first determining module 100 configured to determine a unit to be corrected corresponding to a dose monitoring system;
a second determining module 200 configured to determine a first correction factor based on the number of rays emitted by a nonmetal unit to be activated after neutron activation and the unit to be corrected corresponding to the dose monitoring system, wherein the nonmetal unit to be activated includes a nonmetal component and a first detection part configured to detect the number of rays emitted by the nonmetal component after neutron activation; and
a third determining module 300 configured to correct the dose monitoring system based on the first correction factor, so as to determine a real-time neutron dose of a patient.

In an embodiment of the present disclosure, the second determining module 200 is configured to: set the unit to be corrected as a counting rate to be corrected; determine a first neutron reaction rate based on the number of rays emitted by the nonmetal unit to be activated after neutron activation; and determine the first correction factor based on the counting rate to be corrected and the first neutron reaction rate.

In an embodiment of the present disclosure, the second determining module 200 is further configured to determine a nonmetal average counting rate of an activation period corresponding to the nonmetal unit to be activated based on the counting rate to be corrected; and determine the first correction factor based on the first neutron reaction rate and the nonmetal average counting rate.

In an embodiment of the present disclosure, the neutron dose measurement apparatus further includes a fourth determining module configured to determine a second correction factor based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated includes a metal component and a second detection part configured to detect the number of rays emitted by the metal component after neutron activation; and a fifth determining module configured to correct the first correction factor based on the second correction factor so as to correct the corrected dose monitoring system.

In an embodiment of the present disclosure, the second determining module 200 is further configured to determine a second neutron reaction rate based on the number of rays emitted by the metal unit to be activated after neutron activation; determine a metal average counting rate of the activation period corresponding to the metal unit to be activated based on the counting rate to be corrected; and determine the second correction factor based on the first neutron reaction rate and the metal average counting rate and/or the second neutron reaction rate.

In an embodiment of the present disclosure, the fifth determining module is further configured to determine difference information between the first correction factor and the second correction factor; determine a correction value corresponding to the dose monitoring system based on the first correction factor if the first correction factor is determined to meet a preset difference threshold condition based on the difference information; and determine the real-time neutron dose of the patient based on the correction value corresponding to the dose monitoring system, a dose conversion factor and the irradiated time corresponding to the patient.

In an embodiment of the present disclosure, the fifth determining module is further configured to determine a cancer cell real-time dose-rate correction value corresponding to the dose monitoring system based on the correction value corresponding to the dose monitoring system and a boron dose conversion factor; and determine the real-time neutron dose of the cancer cells in the patient based on the cancer cell real-time dose-rate correction value and the irradiated time.

In an embodiment of the present disclosure, the fifth determining module is further configured to determine a non-cancer cell real-time dose-rate correction value corresponding to the dose monitoring system based on the correction value corresponding to the dose monitoring system and a non-cancer cell dose conversion factor; and determine the real-time neutron dose of the non-cancer cells in the patient based on the non-cancer cell real-time dose-rate correction value and the irradiated time.

It should be understood that the operations and functions of the first determining module 100, the second determining module 200, and the third determining module 300 in the neutron dose measurement apparatus provided in FIG. 8 can refer to the neutron dose measurement methods provided in FIGS. 1 to 7 described above, which will not be described in detail here so as to avoid repetition

Thereafter, an electronic device according to an embodiment of the present disclosure will be described with reference to FIG. 9. FIG. 9 is a schematic structural diagram of the electronic device according to an exemplary embodiment of the present disclosure.

As shown in FIG. 9, the electronic device 50 includes one or more processors 501 and a memory 502.

The processor 501 may be a central processing unit (CPU) or other forms of processing unit with data processing capability and/or instruction execution capability, and may control other components in the electronic device 50 to perform desired functions.

The memory 502 may include one or more computer program products. The computer program products may include various forms of computer-readable storage media, such as a volatile memory and/or a nonvolatile memory. The volatile memory may include, for example, a random access memory (RAM) and/or a cache, etc. The nonvolatile memory may include, for example, a read-only memory (ROM), a hard disk, a flash memory, etc. One or more computer program instructions can be stored on the computer-readable storage medium. The processor 501 can run the program instructions to implement the neutron dose measurement method and/or other desired functions of various embodiments herein described above. Various contents such as the counting rate to be corrected, the first correction factor, the second correction factor and the real-time neutron dose can also be stored in the computer-readable storage medium.

In one example, the electronic device 50 may further include an input device 503 and an output device 504, which are interconnected by a bus system and/or other forms of connection mechanisms (not shown).

The input device 503 may include, for example, a keyboard, a mouse, etc.

The output device 504 can output various information to the outside, including the counting rate to be corrected, the first correction factor, the second correction factor, the real-time neutron dose and so on. The output device 504 may include, for example, a display, a speaker, a printer, a communication network and a remote output device connected thereto.

Definitely for the purpose of simplification, only some components related to the present disclosure in the electronic device 50 are shown in FIG. 9, and components such as a bus, an input/output interface and the like are omitted. In addition, according to the specific application, the electronic device 50 can further include any other suitable components.

In addition to the above methods and devices, embodiments of the present disclosure may also be computer program products including computer program instructions that, when executed by a processor, cause the processor to perform the steps in the neutron dose measurement method according to various embodiments of the present disclosure described above in this specification.

The computer program products can write program codes for performing the operations of the embodiments of the present disclosure in any combination of one or more programming languages. The programming languages include object-oriented programming languages such as Java, C++, etc., and conventional procedural programming languages such as "C" or similar programming languages. The program codes may be executed entirely on the user computing device, partly on the user computing device, as an independent software package, partly on the user computing device and partly on the remote computing device, or entirely on the remote computing device or the server.

In addition, the embodiment of the present disclosure can also be a computer-readable storage medium, on which computer program instructions are stored. The computer program instructions, when executed by a processor, cause the processor to perform the steps in the neutron dose measurement method according to various embodiments of the present disclosure described above in this specification.

The computer-readable storage medium can use any combination of one or more readable media. The readable medium may be a readable signal medium or a readable storage medium. The readable storage medium may include, for example, but not limited to, an electrical, magnetic, optical, electromagnetic, infrared, or semiconductor system, apparatus or device, or any combination of the foregoing. More specific examples (a non-exhaustive list) of the readable storage medium include: an electrical connection with one or more wires, a portable disk, a hard disk, a random access memory (RAM), a read-only memory (ROM), an erasable programmable read-only memory (EPROM or a flash memory), an optical fiber, a portable compact disk read-only memory (CD-ROM), an optical storage device, a magnetic storage device, or any suitable combination of the foregoing.

The basic principle of the present disclosure has been described above in combination with specific embodiments, but it should be pointed out that the advantages, superiorities and effects mentioned in the present disclosure are only examples rather than limitations, and these advantages, superiorities and effects cannot be considered as necessary for all embodiments of the present disclosure. In addition, the specific details disclosed above are only for the purpose of illustration and easy understanding, rather than limitation. The above details do not limit that the present disclosure must be implemented with the above specific details.

The block diagrams of devices, apparatuses, equipment and systems involved in the present disclosure are only illustrative examples and are not intended to require or imply that the devices, apparatuses, equipment and systems must be connected, arranged and configured in the manner shown in the block diagrams. It will be recognized by those skilled in the art that these devices, apparatuses, equipment and systems can be connected, arranged and configured in any manner. Terms such as "including", "containing", "having" and so on are open-ended words, which mean "including but not limited to" and can be used interchangeably. The terms "or" and "and" as used herein refer to the terms "and/or" and can be used interchangeably with each other unless the context clearly dictates otherwise. The term "such as" used here refers to the phrase "such as but not limited to" and can be used interchangeably.

It should also be pointed out that in the apparatus, device and method of the present disclosure, individual components or steps can be decomposed and/or recombined. These decompositions and/or recombinations should be regarded as equivalents of the present disclosure.

The above description of the disclosed aspects is provided to enable those skilled in the art to make or use the present disclosure. Various modifications to these aspects will be apparent to those skilled in the art, and the general principles defined herein can be applied to other aspects without departing from the scope of the present disclosure. Therefore, the present disclosure is not intended to be limited to the aspects shown herein, but conforms to the widest scope consistent with the principles and novel features disclosed herein.

The foregoing description has been presented for purposes of illustration and description. Furthermore, this description is not intended to limit the embodiments of the present disclosure to the forms disclosed herein. Although several example aspects and embodiments have been discussed above, those skilled in the art will recognize certain variations, modifications, changes, additions and subcombinations thereof.

**Claims**

1.  A neutron dose measurement method, comprising:

    determining a unit to be corrected corresponding to a dose monitoring system;
    determining a first correction factor based on a number of rays emitted by a nonmetal unit to be activated after neutron activation and the unit to be corrected corresponding to the dose monitoring system, wherein the nonmetal unit to be activated comprises a nonmetal component and a first detection part configured to detect the number of rays emitted by the nonmetal component after neutron activation; and
    correcting the dose monitoring system based on the first correction factor to determine a real-time neutron dose of a patient.

2.  The neutron dose measurement method according to claim 1, wherein the unit to be corrected is set as a counting rate to be corrected;

    a first neutron reaction rate is determined based on the number of rays emitted by the nonmetal unit to be activated after neutron activation;
    the first correction factor is determined based on the counting rate to be corrected and the first neutron reaction rate.

3.  The neutron dose measurement method according to claim 2, wherein a nonmetal average counting rate of an activation period corresponding to the nonmetal unit to be activated is determined based on the counting rate to be corrected;
    the first correction factor is determined based on the first neutron reaction rate and the nonmetal average counting rate.

4.  The neutron dose measurement method according to any of claims 1 to 3, further comprising determining a second correction factor based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated comprises a metal component and a second detection part configured to detect the number of rays emitted by the metal component after neutron activation; and correcting the first correction factor based on the second correction factor to correct the corrected dose monitoring system.

5.  The neutron dose measurement method according to claim 4, wherein

    a second neutron reaction rate is determined based on the number of rays emitted by the metal unit to be activated after neutron activation;
    a metal average counting rate of the activation period corresponding to the metal unit to be activated is determined based on the counting rate to be corrected;
    the second correction factor is determined based on the first neutron reaction rate and the metal average counting rate and/or the second neutron reaction rate.

6.  The neutron dose measurement method according to claim 4, wherein correcting the first correction factor based on the second correction factor to correct the corrected dose monitoring system comprises:

    determining difference information between the first correction factor and the second correction factor;
    determining a correction value corresponding to the dose monitoring system based on the first correction factor when the first correction factor is determined to meet a preset difference threshold condition based on the difference information;
    determining the real-time neutron dose of the patient based on the correction value corresponding to the dose monitoring system, a dose conversion factor and irradiated time corresponding to the patient.

7.  The neutron dose measurement method according to claim 6, wherein the dose conversion factor comprises a boron dose conversion factor, and wherein determining the real-time neutron dose of the patient based on the correction value corresponding to the dose monitoring system, the dose conversion factor and the irradiated time corresponding to the patient comprises:

determining a cancer cell real-time dose-rate correction value corresponding to the dose monitoring system based on the correction value and the boron dose conversion factor;
determining the real-time neutron dose of cancer cells in the patient based on the cancer cell real-time dose-rate correction value and the irradiated time.

8. The neutron dose measurement method according to claim 6, wherein the dose conversion factor comprises a non-cancer cell dose conversion factor, and wherein determining the real-time neutron dose of the patient based on the correction value corresponding to the dose monitoring system, the dose conversion factor and the irradiated time corresponding to the patient comprises:

determining a non-cancer cell real-time dose-rate correction value corresponding to the dose monitoring system based on the correction value corresponding to the dose monitoring system and the non-cancer cell dose conversion factor;
determining the real-time neutron dose of non-cancer cells in the patient based on the non-cancer cell real-time dose-rate correction value and the irradiated time.

9. The neutron dose measurement method according to claim 1, wherein a material of the nonmetal unit to be activated comprises at least one of phosphorus, sulfur, silicon and bromine.

10. A neutron dose measurement apparatus, comprising:

a first determining module configured to determine a unit to be corrected corresponding to a dose monitoring system;
a second determining module configured to determine a first correction factor based on a number of rays emitted by a nonmetal unit to be activated after neutron activation and the unit to be corrected corresponding to the dose monitoring system, wherein the nonmetal unit to be activated comprises a nonmetal component and a first detection part configured to detect the number of rays emitted by the nonmetal component after neutron activation; and
a third determining module configured to correct the dose monitoring system based on the first correction factor to determine a real-time neutron dose of a patient.

11. The neutron dose measurement apparatus according to claim 10, wherein the second determining module is configured to: set the unit to be corrected as a counting rate to be corrected; determine a first neutron reaction rate based on the number of rays emitted by the nonmetal unit to be activated after neutron activation; and determine the first correction factor based on the counting rate to be corrected and the first neutron reaction rate.

12. The neutron dose measurement apparatus according to claim 11, wherein the second determining module is further configured to determine a nonmetal average counting rate of an activation period corresponding to the nonmetal unit to be activated based on the counting rate to be corrected; and determine the first correction factor based on the first neutron reaction rate and the nonmetal average counting rate.

13. The neutron dose measurement apparatus according to claim 10, further comprising: a fourth determining module configured to determine a second correction factor based on the number of rays emitted by the nonmetal unit to be activated after neutron activation and the unit to be corrected corresponding to the dose monitoring system and/or the number of rays emitted by a metal unit to be activated after neutron activation; wherein the metal unit to be activated comprises a metal component and a second detection part configured to detect the number of rays emitted by the metal component after neutron activation; and a fifth determining module configured to correct the first correction factor based on the second correction factor to correct the corrected dose monitoring system.

14. The neutron dose measurement apparatus according to claim 13, wherein the second determining module is configured to determine a second neutron reaction rate based on the number of rays emitted by the metal unit to be activated after neutron activation; determine a metal average counting rate of the activation period corresponding to the metal unit to be activated based on the counting rate to be corrected; and determine the second correction factor based on the first neutron reaction rate and the metal average counting rate and/or the second neutron reaction rate.

15. The neutron dose measurement apparatus according to claim 13, wherein the fifth determining module is further configured to determine difference information between the first correction factor and the second correction factor;

determine a correction value corresponding to the dose monitoring system based on the first correction factor when the first correction factor is determined to meet a preset difference threshold condition based on the difference information; and determine the real-time neutron dose of the patient based on the correction value corresponding to the dose monitoring system, a dose conversion factor and irradiated time corresponding to the patient.

S10

Determine a unit to be corrected corresponding to a dose monitoring system

S20

Determine a first correction factor based on the number of rays emitted by a nonmetal unit to be activated after neutron activation and the unit to be corrected corresponding to the dose monitoring system

S30

Correct the dose monitoring system based on the first correction factor to determine a real-time neutron dose of a patient

FIG. 1

/ S31

Determine a first neutron reaction rate based on the number of correction rays emitted by the nonmetal unit to be activated after neutron activation

/ S32

Determine the first correction factor based on the counting rate to be corrected and the first neutron reaction rate

FIG. 2

/ S21

Determine a second neutron reaction rate based on the number of rays emitted by the metal unit to be activated after neutron activation

/ S22

Determine a metal average counting rate of an activation period corresponding to the metal unit to be activated based on the counting rate to be corrected

/ S23

Determine the second correction factor based on the first neutron reaction rate and the metal average counting rate and/or the second neutron reaction rate

FIG. 3

y

S41

Determine difference information between the first correction factor and the second correction factor

S43

Determine a correction value corresponding to the dose monitoring system based on the first correction factor if the first correction factor is determined to meet a preset difference threshold condition based on the difference information

S44

Determine the real-time neutron dose of the patient based on the correction value corresponding to the dose monitoring system, a dose conversion factor and the irradiated time corresponding to the patient

FIG. 4

S441

Determine a cancer cell real-time dose-rate correction value corresponding to the dose monitoring system based on the correction value corresponding to the dose monitoring system and the boron dose conversion factor

S442

Determine the real-time neutron dose of the cancer cells in the patient based on the cancer cell real-time dose-rate correction value and the irradiated time

FIG. 5

S443

Determine a non-cancer cell real-time dose-rate correction value corresponding to the dose monitoring system based on the correction value corresponding to the dose monitoring system and the non-cancer cell dose conversion factor

S444

Determine the real-time neutron dose of the non-cancer cells in the patient based on the non-cancer cell real-time dose-rate correction value and the irradiated time

FIG. 6

```
                          ┌──────────────┐         ┌──────────────┐
                          │ Metal unit to │ /4      │ Nonmetal unit │ /9
                          │ be activated  │         │ to be activated│
                          └──────────────┘         └──────────────┘
                                  │                        │
┌──────────────┐                  ▼                        ▼
│ BF₃ detector │ /1      ┌──────────────┐         ┌──────────────┐
└──────────────┘         │  Activation  │ /5      │  Activation  │ /5
        │                │   detector   │         │   detector   │
        ▼                └──────────────┘         └──────────────┘
┌──────────────┐                  │                        │
│   Signal     │ /2               ▼                        ▼
│  processing  │         ┌──────────────┐         ┌──────────────┐
│   circuit    │         │    Signal    │ /6      │    Signal    │ /6
└──────────────┘         │  processing  │         │  processing  │
        │                │   circuit    │         │   circuit    │
        ▼                └──────────────┘         └──────────────┘
┌──────────────┐                  │                        │
│   Counter    │ /3               ▼                        ▼
└──────────────┘         ┌──────────────┐         ┌──────────────┐
                         │    Second    │ /7      │ First neutron │ /10
                         │   neutron    │         │ reaction rate │
                         │ reaction rate│         └──────────────┘
                         └──────────────┘                 │
                                  │                        ▼
                                  ▼                ┌──────────────┐
                         ┌──────────────┐          │First correction│ /11
                         │   Second     │ /8       │    factor     │
                         │  correction  │          └──────────────┘
                         │   factor     │
                         └──────────────┘
```

Determine whether the first correction factor meets the preset difference threshold condition based on the difference information between the first correction factor and the second correction factor — S42

NO

YES

Determine a correction value corresponding to the dose monitoring system based on the first correction factor and the counting rate to be corrected — S43

Determine the real-time neutron dose of the patient based on the correction value corresponding to the dose monitoring system, the dose conversion factor and the irradiated time corresponding to the patient — S44

FIG. 7

```
                                    /100
   ┌─────────────────────────────────────┐
   │       First determining module      │
   └─────────────────────────────────────┘
                    │           /200
   ┌─────────────────────────────────────┐
   │      Second determining module      │
   └─────────────────────────────────────┘
                    │           /300
   ┌─────────────────────────────────────┐
   │       Third determining module      │
   └─────────────────────────────────────┘
```

FIG. 8

```
   ┌──────────────────────────────────────────────────────┐
   │              ┌────────────────────┐                   │
   │              │   Processor 501    │                   │
   │              └────────────────────┘                   │
   │                        │                              │
   │      ┌─────────────────┼──────────────────┐           │
   │      │                 │                  │           │
   │ ┌──────────┐   ┌──────────────┐   ┌────────────────┐  │
   │ │  Input   │   │  Memory 502  │   │  Output device │  │
   │ │device 503│   │              │   │      504       │  │
   │ └──────────┘   └──────────────┘   └────────────────┘  │
   │                              Electronic device 50     │
   └──────────────────────────────────────────────────────┘
```

FIG. 9